# EUROPÄISCHE PATENTSCHRIFT

(11) **EP 2 257 248 B1**
(45) Veröffentlichungstag und Bekanntmachung des Hinweises auf die Patenterteilung: **12.10.2011**
(21) Anmeldenummer: 09713273.2
(22) Anmeldetag: 12.02.2009
(51) Int. Cl.: A61F 2/90, A61F 2/00, A61F 2/82

(54) **STENT UND VERFAHREN ZUM HERSTELLEN EINES DERARTIGEN STENTS**
STENT AND METHOD FOR THE PRODUCTION OF SUCH A STENT
ENDOPROTHÈSE VASCULAIRE ET SON PROCÉDÉ DE FABRICATION

(30) Priorität: 22.02.2008 DE 102008010507
(43) Veröffentlichungstag der Anmeldung: 08.12.2010
(73) Patentinhaber: Acandis GmbH & Co. KG, 76327 Pfinztal (DE)
(72) Erfinder: CATTANEO, Giorgio, 76199 Karlsruhe (DE); SCHÜSSLER, Kirsi, 76327 Pfinztal (DE)
(74) Vertreter: Kilchert, Jochen
(86) Internationale Anmeldenummer: PCT/EP2009/000996
(87) Internationale Veröffentlichungsnummer: WO 2009/103457

(56) Entgegenhaltungen:
- WO-A-2006/125022
- US-A1- 2002 107 562
- US-A1- 2007 239 261

## Beschreibung

Die Erfindung betrifft einen Stent gemäß dem Oberbegriff des Anspruchs 1 sowie ein Verfahren zum Herstellen eines Stents. Ein derartiger Stent ist beispielsweise aus der US 6,428,569 bekannt.

Zum Aufweiten und Abstützen von Blutgefäßen werden Stents eingesetzt, die aus einer rohrförmigen Gitterstruktur gebildet sind. Auf diese Weise werden insbesondere Stenosen oder Aneurysmen behandelt. Stenosen, d.h. Verengungen eines Blutgefäßes, führen stromabwärts zu einer Minderversorgung des Gewebes mit Nährstoffen. Mit Hilfe eines Stents wird die Engstelle aufgeweitet und stabilisiert, so dass eine ausreichende Blutströmung gewährleistet ist. Aneurysmen stellen Aussackungen von Blutgefäßen dar, die zu einer starken Beanspruchung der Blutgefäßwände führen. Diese Aussackungen können mit der Zeit wachsen und ein Aufreißen der Gefäßwand bewirken. Durch Platzierung eines geeigneten Stent im Bereich eines Aneurysmas in einem Blutgefäß wird die Blutströmung im Aneurysma reduziert bzw. gestoppt, so dass innerhalb des Aneurysmas eine Gerinnung des Blutes erfolgt, wodurch ein weiteres Wachstum des Aneurysmas verhindert wird.

Aus WO2006/125022 ist ein Stent zur Behandhung eines Aneurysmas betannt. In bestimmten Ausführungen sind die Stege des Stents mit Kontaktelementen versechen, die daran angepasst sind, das Aneurysma von der Blutströmung abzutrennen, indem die Kontaktelemente breiter als die zugeordneten Stege sind.

Ein Stent übt während und nach der Implantation eine radiale Kraft auf die anliegende Blutgefäßwand aus, die sich über den Umfang des Stents auf die Anzahl der Stege verteilt. Bei einer relativ geringen Anzahl von Stegen übertragen diese jeweils eine hohe Kraft auf die Blutgefäßwand, was unerwünscht ist, da es dadurch zu einer Reizung bzw. Verletzung des Blutgefäßes kommen kann. Durch die resultierende Entzündungsreaktion erhöht sich das Risiko einer Restenose, d.h. einer erneuten Verengung des Blutgefäßes, nachdem dieses durch einen Stent aufgeweitet wurde. Daher wird in der auf die Anmelderin zurückgehenden, nachveröffentlichten deutschen Patentanmeldung Nr. 10 2007 019 772 vorgeschlagen, die Anzahl der Stege zu erhöhen, so dass die auf die Gefäßwand wirkenden Kräfte besser verteilt werden und somit jeder einzelne Steg eine geringere Kraft auf die Gefäßwand ausübt.

In der eingangs genannten US 6,428,569 ist ein Stent mit einer feinmaschigen Gitterstruktur beschrieben, deren Stege eine sehr geringe Breite aufweisen. Die sich aus der geringen Stegbreite ergebende kleine Kontaktfläche zwischen dem einzelnen Steg und der Gefäßwand führt dazu, dass der lokale Druck, der pro Steg bei einer bestimmten Radialkraft auf die Gefäßwand ausgeübt wird, relativ groß ist.

Die Breite und Anzahl der Stege kann allerdings nicht beliebig erhöht werden, ohne die Implantierbarkeit des Stents zu beeinträchtigen. Um den Stent zu implantieren, wird dieser komprimiert und innerhalb eines Katheters platziert, mit dessen Hilfe der Stent in das Blutgefäß eingeführt wird. Die maximale Stegbreite bzw. die maximale Steganzahl wird durch die geometrischen Randbedingungen begrenzt, die durch die Größe des Katheters bzw. die Größe der zu behandelnden Blutgefäße gesetzt sind.

Hinzu kommt, dass die Kraftübertragung nicht gleichmäßig über die gesamte Außenfläche der Stege erfolgt, da die Stege aufgrund des hohen lokalen Drucks in die Gefäßwand einschneiden. Dadurch wird die Radialkraft im Bereich der Längskanten der Stege stärker auf die Gefäßwand übertragen, als im Innenbereich der Stege. Die sich daraus ergebende Spannungskonzentration im Bereich der Längskanten erhöht die Gefahr der Restenose.

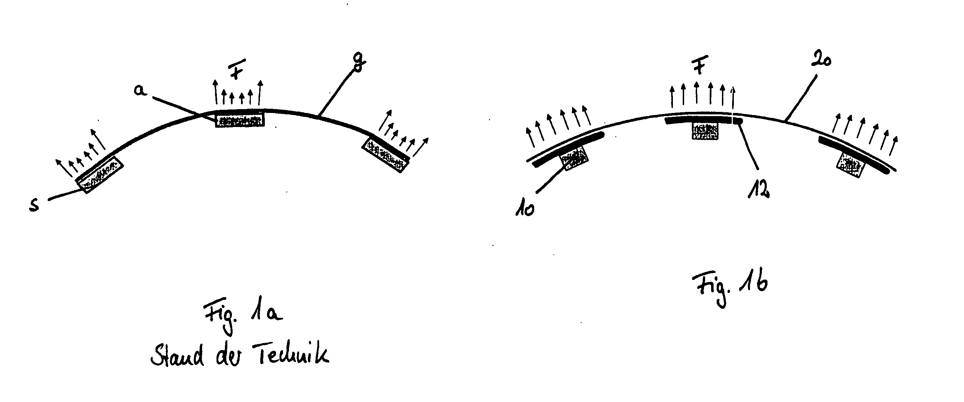

Dieser Effekt, der mit größerer Stegbreite zunimmt, ist beispielhaft in Fig. 1a dargestellt, die einen Teilquerschnitt durch einen Stent gemäß dem Stand der Technik im implantierten Zustand zeigt. Die Stege s liegen an einer Gefäßwand g an und übertragen eine, durch das Aufweiten des Stents hervorgerufenen Radialkraft F auf die Gefäßwand g. In Fig. 1a ist die Radialkraft F durch Pfeile dargestellt, wobei die Größe der Kraft jeweils durch die Länge des Pfeils wiedergegeben ist. Durch den hohen lokalen Druck, den die Stege s auf die Gefäßwand g übertragen, wird diese gespannt und verformt, wobei die größte Verformung im Bereich der Längskanten a der Stege s auftritt. Das führt dazu, dass die Krafteinleitung hauptsächlich in den Randbereichen bzw. an den Längskanten a der Stege s erfolgt.

Insgesamt sind also zwei konstruktive Maßnahmen zielführend, um die Belastungen auf eine Blutgefäßwand durch einen Stent zu reduzieren. Einerseits wird durch eine erhöhte Anzahl von Stegen über den Querschnitt des Stents die homogene Kraftverteilung auf die Gefäßwand verbessert, so dass die lokalen Kräfte pro Steg reduziert werden. Andererseits wird eine Reduzierung der lokalen Drücke, die im Bereich der einzelnen Stege auf die Gefäßwand wirken, dadurch erreicht, dass die Stegbreite und somit die Kontaktfläche erhöht wird. Die Kombination beider Maßnahmen, d.h. die Erhöhung der Steganzahl bei gleichzeitiger Vergrößerung der Stegbreite, ist durch die geometrischen Beschränkungen bei der Implantation begrenzt.

Der Erfindung liegt daher die Aufgabe zu Grunde, einen Stent mit einer rohrförmigen Gitterstruktur anzugeben, der die Belastung der Gefäßwände und das Risiko einer Restenose verringert, ohne dabei die Implantierbarkeit des Stents signifikant zu beeinträchtigen. Ferner liegt der Erfindung die Aufgabe zu Grunde, ein Verfahren zum Herstellen eines derartigen Stents anzugeben.

Erfindungsgemäß wird diese Aufgabe im Hinblick auf den Stent durch den Gegenstand des Patentanspruchs 1 und im Hinblick auf das Verfahren durch den Gegenstand des Patentanspruchs 22 gelöst.

Der Erfindung liegt der Gedanke zu Grunde, einen Stent mit einer rohrförmigen Gitterstruktur anzugeben, die Gitterelemente, insbesondere Stege, und durch Gitterelemente begrenzte Zellen umfasst, wobei die Gitterstruktur in einen komprimierten Zustand mit relativ kleinerem Querschnittsdurchmesser und in einen expandierten Zustand mit relativ größerem Querschnittsdurchmesser überführbar ist. Den Gitterelementen sind dabei flexible Kontaktelemente zugeordnet, die zur Übertragung von Radialkräften auf eine Gefäßwand angepasst sind und sich auf einem Außenumfang der Gitterstruktur im Wesentlichen in Längsrichtung der jeweils zugeordneten Gitterelemente erstrecken, wobei die Kontaktelemente zumindest abschnittsweise breiter als die jeweils zugeordneten Gitterelemente sind.

Der erfindungsgemäße Stent ermöglicht im Vergleich zum Stand der Technik die Anordnung vieler Stege mit, im expandierten Zustand, großen Kontaktflächen zwischen den Stegen und der Gefäßwand, wobei der Stent zudem gut implantierbar ist.

Hierzu sieht die Erfindung auf dem Außenumfang des Stents angeordnete Kontaktelemente vor, die den Gitterelementen zugeordnet sind. Die Kontaktelemente sind breiter als die Gitterelemente. Dadurch wird im implantierten bzw. expandierten Zustand eine im Vergleich zum einfachen Gitterelement vergrößerte Kontaktfläche zwischen Stent und Gefäßwand erreicht. Die zumindest abschnittsweise breiteren Kontaktelemente bedeuten, dass diese zumindest im expandierten Zustand des Stents über wenigstens eine Kante des jeweils zugeordneten Gitterelementes vorstehen und damit die durch das Gitterelement gebildete Kontaktfläche zwischen Gitterelement bzw. insgesamt zwischen Stent und Gefäßwand vergrößern. Unter der Breite eines Kontaktelements wird in erster Linie die Erstreckung des Kontaktelements im Wesentlichen senkrecht zu seiner Längserstreckung angesehen. Die Erfindung ist hierauf nicht eingeschränkt, sondern umfasst generell Kontaktelemente, die eine Vergrößerung der Kontaktfläche im expandierten Zustand im Vergleich zur Kontaktfläche im komprimierten Zustand ermöglichen.

Die gute Implantierbarkeit des erfindungsgemäßen Stents bleibt trotz der vergrößerten Kontaktfläche erhalten, da die Kontaktelemente flexibel sind und sich beim Crimpen bzw. Komprimieren des Stents verformen können. Deshalb können mit dem erfindungsgemäßen Stent ausreichend kleine Durchmesser erreicht werden, um diesen mit Hilfe üblicher Zuführsysteme bzw. Katheter zu implantieren. Bei der Implantation spannen sich die flexiblen Kontaktelemente durch die Bewegung der Gitterelemente und/oder durch eine Eigenbewegung bei der Verwendung eines Formgedächtnismaterials auf und nehmen Ihre Wirkposition ein. In der Wirkposition werden die Kontaktelemente durch die Gitterelemente und/oder durch eine entsprechende Konditionierung gespannt und bilden die Kontaktfläche, über die die Radialkraft auf die Gefäßwand übertragen wird.

Aufgrund der reversibel vergrößerbaren Kontaktfläche, die durch die flexiblen Kontaktelemente erreichbar ist, kann die Breite der Gitterelemente verringert werden, ohne dass der lokale, von den einzelnen Gitterelementen auf die Gefäßwand ausgeübte Druck steigt.

Auf diese Weise kann die Kontaktfläche zwischen Stent und Gefäßwand in Verbindung mit einer hohen Anzahl von Gitterelementen erhöht werden, da bei dem erfindungsgemäßen Stent die Breite des einzelnen Gitterelements verringert werden kann, ohne dass sich dadurch die im implantierten Zustand wirksame Kontaktfläche verkleinert.

Insgesamt ermöglicht der erfindungsgemäße Stent durch die flexiblen Kontaktelemente eine veränderliche Kontaktfläche zur Übertragung der Radialkraft, die sich im komprimierten Zustand der Fläche der Gitterelemente annähert und sich im expandierten Zustand vergrößert.

Ein weiterer Vorteil des erfindungsgemäßen Stents besteht darin, dass sich die flexiblen Kontaktelemente besser als die starren Stege an die gekrümmte Gefäßwand anpassen, so dass die bei starren Stegen nachteiligen Spannungsspitzen entlang der Längskanten vermieden werden.

Für die Behandlung von Stenosen innerhalb eines Blutgefäßes ist der erfindungsgemäße Stent besonders effektiv, da durch die große Kontaktfläche zwischen den flexiblen Kontaktelementen und der Gefäßwand die Belastung des Blutgefäßes und das Risiko einer Verletzung verringert wird. Ferner wird mit Hilfe des erfindungsgemäßen Stents auch die Behandlung von Aneurysmen verbessert, da das Aneurysma durch den großen Anteil der geschlossenen Umfangsfläche des Stents strömungstechnisch stark vom Blutfluss im Blutgefäß abgekoppelt wird. Dadurch wird die Gerinnung des Blutes im Aneurysma gefördert und ein Bruch der Aneurysmawand verhindert.

Der erfindungsgemäße Stents ist zur Behandlung von vulnerablen Plaques bzw. Softplaques geeignet. Dabei handelt es sich um eine Ansammlung von weichem Gewebe innerhalb einer Blutgefäßwand. Im Gegensatz zu Stenosen kommt es dabei nicht zu einer Verengung des Gefäßdurchmessers. Trotzdem besteht die Gefahr von Gefäßverschlüssen, da das weiche Gewebe innerhalb der Gefäßwand lediglich durch eine dünne Membran von der Blutbahn getrennt wird. Eine Verletzung dieser dünnen Membran kann zu einer Ablösung des Plaques führen, wodurch es stromabwärts zum Verschluss kleinerer Gefäße kommen kann. Mit Hilfe des erfindungsgemäßen Stents kann die dünne Membran eines Softplaques an der Gefäßwand stabilisiert werden, ohne dass die über die Stege übertragenen Kräfte, im Gegensatz zu bekannten Stents, zu einer Verletzung der Membran führen. Erfindungsgemäße Stents können auch zur Behandlung von härteren Plaques, beispielsweise Kalziumablagerungen in Blutgefäßen, eingesetzt werden. Durch die Minimierung der lokalen Kräfte im Bereich der Gitterelemente wird bei härteren Plaques vermieden, dass Ablagerungen, beispielsweise Kalziumablagerungen, durchtrennt und partikelweise mit dem Blutstrom in kleinere Gefäße transportiert werden.

Vorzugsweise bilden die Gitterelemente jeweils zusammen mit einem zugeordneten Kontaktelement zumindest abschnittsweise einen im Wesentlichen T-förmigen oder L-förmigen Querschnitt. Bei dem T-förmigen Querschnitt stehen die Kontaktelemente über beide Seiten der zugeordneten Gitterelemente vor, insbesondere symmetrisch vor. Bei dem L-förmigen Querschnitt stehen die Kontaktelemente nur über eine Seite der zugeordneten Gitterelemente vor.

Die Kontaktelemente können jeweils um wenigstens 10 %, insbesondere wenigstens 15 %, insbesondere wenigstens 20 %, insbesondere wenigstens 30 %, insbesondere wenigstens 40 %, insbesondere wenigstens 50 %, insbesondere wenigstens 60 %, insbesondere wenigstens 70 %, insbesondere wenigstens 80 %, insbesondere wenigstens 90 %, insbesondere wenigstens 100%, breiter sein, als das jeweils zugeordnete Gitterelement. Vorzugsweise sind die Kontaktelemente nicht mehr als 500 %, insbesondere höchstens 400 %, insbesondere höchstens 300 %, insbesondere höchstens 200 %, insbesondere höchstens 100 %, breiter als das jeweilige Gitterelement. Auf diese Weise wird eine ausreichende Erhöhung der Kontaktfläche zwischen Stent-Außenfläche und Gefäßwand-Innenfläche erreicht, so dass die Belastung der Gefäßwand durch die auftretenden Radialdrücke lokal, d.h. im Bereich der Gitterelemente, minimiert wird.

Generell bezieht sich die Angabe der Breite der Kontaktelemente auf den Bereich bzw. Abschnitt des Kontaktelements, der gegenüber dem Gitterelement die größte Breite aufweist bzw. am weitesten über die Breite des Gitterelements hinausragt. Dies gilt sowohl für Kontaktelemente, die beidseitig (T-Form), als auch einseitig (L-Form) über das jeweils zugeordnete Gitterelement hinausragen.

Im Falle des beidseitigen Überragens können die über das Gitterelement vorstehenden Seiten des Kontaktelements unterschiedlich weit über das Gitterelement hinausragen. Der Abstand der einen Längskante des Kontaktelements zum Gitterelement kann kleiner oder größer sein als der Abstand der anderen Längskante zum Gitterelement. Der Abstand der beiden Längskanten zueinander kann insgesamt größer sein, als die Breite des zugeordneten Gitterelements in diesem Bereich. Der Abstand zwischen einer Längskante des Kontaktelements und dem Gitterelement kann entlang des Gitterelements variieren.

Ferner kann die Breite eines Kontaktelements oder mehrerer Kontaktelemente über den Umfang und/oder die Länge des Stents variabel sein. Das bedeutet, dass sich die Breite eines Kontaktelements in Umfangs- und/oder in Längsrichtung des Stents ändert. Dasselbe gilt für die Breite mehrerer Kontaktelemente. Dadurch können Bereiche mit unterschiedlicher Flexibilität oder unterschiedlicher Festigkeit eingestellt werden. Ferner kann der Stent lokal an zu behandelnde Stellen der Gefäßwand angepasst werden. Beispielsweise können die Kontaktelemente in Bereichen des Stents breiter sein, die nach der Implantation ein Aneurysma abdecken, so dass die relativ breiteren Kontaktelemente das Aneurysma effizient von der Blutströmung im Gefäß abtrennen und somit positiv auf die Strömungsverhältnisse innerhalb des Aneurysmas einwirken. Die Kontaktelemente können beispielsweise in Bereichen des Stents relativ schmaler sein, die nach der Implantation im Bereich von Gefäßverzweigungen angeordnet sind, so dass eine ausreichende Blutströmung in das abzweigende Gefäß gewährleistet ist.

Bei einer bevorzugten Ausführungsform des erfindungsgemäßen Stents weisen die Zellen der Gitterstruktur jeweils eine Öffnung auf, die durch die Kontaktelemente derjenigen Gitterelemente begrenzt ist, die die jeweilige Zelle bilden. Die Öffnungen sind dabei auf dem Außenumfang der Gitterstruktur angeordnet und im implantierten Zustand der Gefäßinnenwand zugewandt. Die Öffnungen können jeweils an die Kontur der Zelle angepasst sein. Vorzugsweise sind die Öffnungen im Wesentlichen rautenförmig. Die Rautenform ermöglicht in besonders vorteilhafter Weise die Überführung der Gitterstruktur in einen komprimierten bzw. expandierten Zustand. Das ist insbesondere dann der Fall, wenn die durch die Gitterelemente begrenzten Zellen ebenfalls eine im Wesentlichen rautenförmige Struktur aufweisen, wobei die Erfindung nicht darauf beschränkt ist, sondern auch Stents mit einer offenen Zellstruktur umfasst. Die Gitterstruktur mit rautenförmigen Öffnungen kann besonders einfach in den komprimierten Zustand überführt werden, indem die in Längsrichtung der Gitterstruktur ausgerichtete Diagonale der Rautenform verlängert und die in Umfangsrichtung ausgerichtete Diagonale der Rautenform verkürzt wird. Andererseits bewirkt eine Verlängerung der in Umfangsrichtung ausgerichteten Diagonale der Rautenform bei gleichzeitiger Verkürzung der in Längsrichtung der Gitterstruktur ausgerichteten Diagonale der Rautenform eine Expansion des Stents.

Bei einer weiteren bevorzugten Ausführungsform weisen die Kontaktelemente jeweils wenigstens eine Längskante auf, die sich parallel zu einer Längsachse des jeweils zugeordneten Gitterelements erstreckt. Ferner können die Kontaktelemente jeweils wenigstens eine Längskante aufweisen, die in einem Winkel zur Längsachse des jeweils zugeordneten Gitterelements angeordnet ist. Generell gilt, dass die Breite eines Kontaktelements und/oder der Abstand zwischen einer Längskante des Kontaktelements und dem zugeordneten Gitterelement entlang des Gitterelements variieren können. Beispielsweise kann das Kontaktelement in einem Bereich des Gitterelements schmaler sein als in einem anderen Bereich des Gitterelements bzw. Stegs. Insbesondere kann beispielsweise ein einem Gitterelement zugeordnetes Kontaktelement an einem ersten axialen Ende des Gitterelements genau so breit und an einem zweiten axialen Ende beispielsweise doppelt so breit oder breiter wie das Gitterelement sein. Dabei kann das Kontaktelement am ersten Ende mit dem Gitterelement abschließen (Abstand = 0) und am zweiten Ende um einen bestimmten Betrag, bspw. um die Breite des Gitterelements, über dieses vorstehen.

Des Weiteren können die Kontaktelemente jeweils zwei Längskanten aufweisen, die zueinander parallel und/oder zu einer Längsachse des jeweils zugeordneten Gitterelements in einem Winkel angeordnet sind. Die Kontaktelemente können im Wesentlichen rechteckige Streifen bilden, die im Wesentlichen diagonal zum jeweils zugeordneten Gitterelement angeordnet sind. Dadurch wird erreicht, dass Belastungen bzw. Beschädigungen der Kontaktelemente durch das Überführen der Gitterstruktur in einen komprimierten bzw. expandierten Zustand verringert werden, da durch geeignete Wahl des Winkels zwischen Kontaktelement und Gitterelement eine Streckung bzw. Dehnung des Kontaktelements beim Komprimieren bzw. Expandieren der Gitterstruktur vermieden wird. Der geeignete Winkel hängt vor allem von der jeweiligen Stentgeometrie und dem gewünschten Verhältnis der Querschnittsdurchmesser im expandierten und komprimierten Zustand ab und kann empirisch, beispielsweise durch Versuche, bestimmt werden.

Bei einer bevorzugten Ausführungsform sind die Kontaktelemente im Bereich von Eckpunkten der Zellen im expandierten Zustand überlappend und/oder aneinander angrenzend angeordnet oder miteinander verbunden. Durch die überlappende oder angrenzende Anordnung der Kontaktelemente im Bereich von Eckpunkten der Zellen wird erreicht, dass die Kontaktelemente bei der Überführung der Gitterstruktur in einen komprimierten bzw. expandierten Zustand gegeneinander verschieblich sind, so dass eine übermäßige Beanspruchung, insbesondere Dehnung, der Kontaktelemente durch Bewegung der Gitterstruktur vermieden wird. Besonders vorteilhaft kann die Kombination von überlappenden Kontaktelementen und aneinander angrenzenden Kontaktelementen sein. Bei einer rautenförmigen Struktur der Zellen können beispielsweise die in Umfangsrichtung der Gitterstruktur gegenüber liegenden Eckpunkte der Zellen aneinander angrenzende und die in Längsrichtung der Gitterstruktur gegenüber liegenden Eckpunkte überlappende Kontaktelemente aufweisen. Bei Überführung der Gitterstruktur in einen komprimierten Zustand vergrößert sich der Abstand der in Längsrichtung der Gitterstruktur sich gegenüber liegenden Eckpunkte, so dass die Kontaktelemente im Bereich der in Umfangsrichtung gegenüber liegenden Eckpunkte auseinander gezogen werden. Wenn die Kontaktelemente dort aneinander angrenzend angeordnet sind, können die Kontaktelemente auseinander gleiten. Eine Dehnung der Kontaktelemente wird dadurch vermieden. Andererseits können die an den in Längsrichtung gegenüber liegenden Eckpunkten angeordneten Kontaktelemente überlappen, so dass sich die Kontaktelemente bei der Komprimierung der Gitterstruktur nicht falten, sondern übereinander schieben.

Vorzugsweise weisen die Kontaktelemente eine Höhe bzw. Wandstärke zwischen 0,5 µm und 100 µm, insbesondere 1 µm und 50 µm, insbesondere 2 µm und 30 µm, insbesondere 3 µm und 20 µm, insbesondere 5 µm und 10 µm, auf. Die genannten Höhen bzw. Wandstärken der Kontaktelemente gewährleisten eine hohe Stabilität und gleichzeitig ein geringes Gewicht, so dass einerseits das Blutgefäß ausreichend gestützt und andererseits eine durch die Gewichtskraft des Stents bewirkte zusätzliche Belastung des Blutgefäßes reduziert wird.

Die Höhe der Kontaktelemente entspricht vorzugsweise wenigstens 0,5 %, insbesondere wenigstens 1 %, insbesondere wenigstens 2 %, insbesondere wenigstens 5 %, insbesondere wenigstens 10%, der Höhe des jeweils zugeordneten Gitterelements. Besonders bevorzugt entspricht die Höhe der Kontaktelemente höchstens 50%, insbesondere höchstens 40%, insbesondere höchstens 30%, insbesondere höchstens 20%, insbesondere höchstens 15 %, insbesondere höchstens 10%, der Höhe der Gitterelemente. Dementsprechend kann das Verhältnis der Höhe der Kontaktelemente (KE) zur Höhe des jeweils zugeordneten Gitterelements (GE), d.h. KE:GE, beispielsweise wenigstens 1:20 und höchstens 1:10 betragen. Die Höhe der Gitterelemente variiert je nach Anwendungsfall des einzusetzenden Stents, insbesondere in Abhängigkeit des gewünschten Einsatzortes.

Die Höhe eines oder mehrerer Kontaktelemente kann sowohl über den Umfang und/oder die Länge des Stents als auch über die Breite und/oder die Länge des bzw. der Kontaktelemente variabel sein. Die Konzeption eines Stents mit unterschiedlich hohen Kontaktelementen bzw. mit Kontaktelementen, die verschiedene Wandstärken aufweisen, ermöglicht bereichsweise unterschiedliche Festigkeits- und Flexibilitätseigenschaften des Stents. Beispielsweise kann der Stent abschnittsweise mit dünneren Kontaktelementen versehen werden, so dass sich der Stent im implantierten Zustand flexibel an eine Gefäßkrümmung oder -verzweigung anpasst. Die Einstellung unterschiedlicher Wandstärken kann innerhalb eines einzelnen Kontaktelements und/oder bei mehreren Kontaktelementen erfolgen.

Bei einer bevorzugten Ausführungsform weisen die Kontaktelemente im Bereich des jeweils zugeordneten Gitterelements eine größere Höhe bzw. Wandstärke auf als in einem Außenbereich, d.h. in einem von dem jeweils zugeordneten Gitterelement entfernten Bereich bzw. in dem Bereich der Kontaktelemente, der über das jeweils zugeordnete Gitterelement hinausragt. Auf diese Weise wird die Krafteinleitung in die Gefäßwand verbessert, insbesondere gleichmäßiger verteilt, da der Übergang von einem Bereich, in dem die Kraft in die Gefäßwand eingeleitet wird, zu einem Bereich, in dem weniger bzw. keine Kräfte übertragen werden (Außenbereich bzw. von Kontaktelementen begrenzte Öffnung) kontinuierlich ist. Erhöhte lokale Drücke an den Außenbereichen bzw. Längskanten der Kontaktelemente werden somit effizient vermieden.

Des Weiteren können die Kontaktelemente ein Formgedächtnismaterial, insbesondere eine Nickel-Titan-Legierung, umfassen. Die gewünschte Form der Kontaktelemente im expandierten bzw. implantierten Zustand kann auf diese Weise bereits bei der Herstellung der Kontaktelemente festgelegt werden. Unter dem Einfluss bestimmter Umgebungsbedingungen, insbesondere der Körpertemperatur, nehmen die Kontaktelemente ihre bei der Herstellung aufgeprägte Form ein, wodurch die Implantation des Stents vereinfacht wird. Besonders vorteilhaft ist die Verwendung von Formgedächtnismaterialien für die Kontaktelemente, wenn auch die Gitterstruktur bzw. die Gitterelemente aus einem Formgedächtnismaterial hergestellt sind. Auf diese Weise ist es möglich, den erfindungsgemäßen Stent in einem komprimierten Zustand in ein Blutgefäß einzubringen, wo der Stent aufgrund der Körpertemperatur innerhalb des Blutgefäßes selbsttätig expandiert und so ohne eine zusätzliche, mechanische Krafteinwirkung beispielsweise eine Stenose aufweitet.

Generell kann durch geeignete Auswahl des Materials, beispielsweise eine Nickel-Titan-Legierung oder eine Polymer-Verbindung, und der Wandstärke der Kontaktelemente ein optimaler Kompromiss zwischen Flexibilität und Festigkeit der Kontaktelemente erreicht werden, so dass die Kontaktelemente sich im implantierten Zustand einerseits gut an die Kontur der Gefäßwand anpassen und andererseits die auftretenden Radialkräfte im Wesentlichen über die gesamte Außenfläche gleichmäßig verteilt übertragen. Die Materialauswahl und die Auslegung der Dimensionen der Kontaktelemente bestimmt der Fachmann dabei anhand des jeweiligen Anwendungsfalls bzw. Einsatzzwecks des Stents. Im Allgemeinen ist die Auswahl vom gewünschten Durchmesser des Stents im expandierten bzw. implantierten Zustand abhängig und kann vom Fachmann beispielsweise empirisch anhand von Versuchen getroffen werden. Dabei kann auch die Verwendung von biodegradierbaren Materialien, wie beispielsweise Magnesium oder Magnesium-Legierungen, in Betracht gezogen werden.

Besonders bevorzugt weisen die Kontaktelemente eine strukturierte Oberfläche, insbesondere Poren oder Rillen, auf. Eine strukturierte Oberfläche fördert die Endothelialisierung, d.h. das Wachstum von gefäßähnlichen Zellen auf der strukturierten Oberfläche, die beispielsweise bei der Behandlung von Aneurysmen zu einem Verschluss des Aneurysmaeingangs bzw. Aneurysmahalses führen. Ferner kann die strukturierte Oberfläche als Depot für medizinisch wirksame Stoffe genutzt werden, beispielsweise Stammzellen, Gentherapeutika, Antikoagulanzien oder andere Stoffe. Gegenüber bekannten Stents wird dabei durch die breiteren Kontaktelemente eine größere Abgabefläche für derartige Stoffe bereitgestellt.

Des Weiteren liegt der Erfindung der Gedanke zu Grunde, ein Verfahren zum Herstellen eines erfindungsgemäßen Stents anzugeben, bei dem flexible Kontaktelemente mit Gitterelementen verbunden oder zusammen mit den Gitterelementen einstückig hergestellt werden derart, dass einem Gitterelement jeweils wenigstens ein Kontaktelement zugeordnet ist, das über den Querschnitt des Gitterelements hinausragt. Falls die flexiblen Kontaktelemente mit den Gitterelementen verbunden werden, erfolgt dies in bevorzugter Weise durch Laserstrahl-Mikroschweißen oder durch Verkleben. Zur Herstellung der Kontaktelemente kommt vorzugsweise ein Sputterverfahren, insbesondere Magnetronsputtern oder Ionenstrahlsputtern, zum Einsatz, wobei die Kontaktelemente entweder direkt auf die Gitterelemente oder mit den Gitterelementen einstückig gesputtert werden können. Das erfindungsgemäße Verfahren ermöglicht die Realisierung einer großen Kontaktfläche zwischen Stent und Gefäßwand im implantierten bzw. expandierten Zustand, wobei eine große Anzahl von Stegen auf dem Umfang des Stents bereitgestellt wird, so dass die auf die Gefäßwand wirkenden Radialkräfte des Stents gleichmäßig auf den Umfang verteilt sind.

Die Erfindung wird im Folgenden anhand von Ausführungsbeispielen unter Bezug auf die beigefügten schematischen Zeichnungen näher erläutert. Darin zeigen
- Fig. 1a: einen Teilquerschnitt durch einen Stent gemäß dem Stand der Technik im implantierten Zustand;
- Fig. 1b: einen Teilquerschnitt durch einen Stent nach einem erfindungsgemäßen Ausführungsbeispiel im implantierten Zustand;
- Fig. 2a: einen Querschnitt durch einen Stent nach einem erfindungsgemäßen Ausführungsbeispiel im implantierten Zustand;
- Fig. 2b: einen Querschnitt durch zwei benachbarte Stege eines Stents nach einem erfindungsgemäßen Ausführungsbeispiel im implantierten Zustand;
- Fig. 3a: einen Querschnitt durch einen Stent nach einem erfindungsgemäßen Ausführungsbeispiel im komprimierten Zustand;
- Fig. 3b: einen Querschnitt durch zwei benachbarte Stege eines Stents nach einem erfindungsgemäßen Ausführungsbeispiel im komprimierten Zustand;
- Fig. 4a-e: jeweils einen Querschnitt durch zwei benachbarte Stege eines Stents nach einem erfindungsgemäßen Ausführungsbeispiel im komprimierten Zustand; und
- Fig. 5a-d: jeweils eine Draufsicht mehrerer benachbarter Zellen nach verschiedenen erfindungsgemäßen Ausführungsbeispielen im expandierten Zustand.

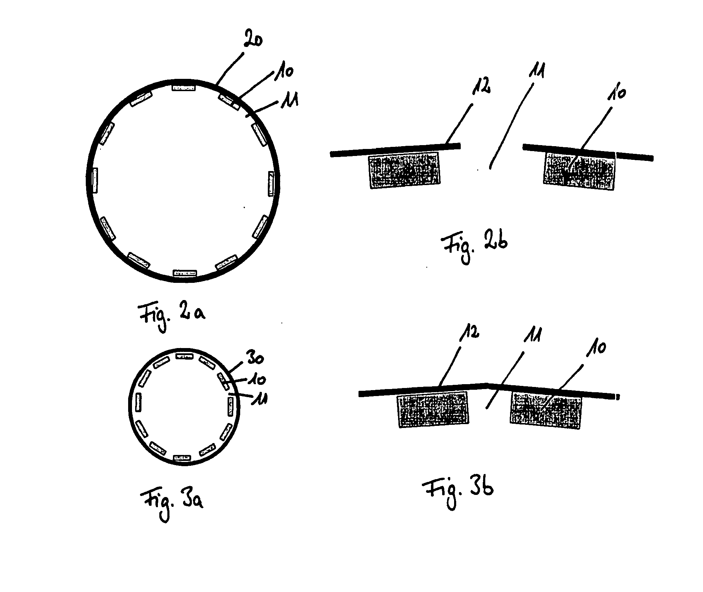

Fig. 1b zeigt einen Teilquerschnitt durch einen erfindungsgemäßen Stent, wobei drei benachbarte Gitterelemente bzw. Stege 10 dargestellt sind, denen jeweils ein Kontaktelement 12 zugeordnet ist, wie auch in Fig. 2b gut zu erkennen. Unter einem Steg wird dabei ein längliches konstruktives Element eines Stents verstanden, der an Nachbarstegen verbunden ist und durch die relative Bewegung zu diesen die Verformung des Stents ermöglicht. Das Kontaktelement 12 hat die Funktion, im implantierten Zustand Radialkräfte vom Stent auf die anliegende Gefäßwand flächig zu übertragen. Dazu weist das Kontaktelement 12 eine durchgängige Oberfläche auf bzw. ist als Flächenelement ausgebildet. Konkret umfasst das Kontaktelement 12 eine Folie, insbesondere eine Metallfolie oder eine Polymerfolie. Andere Mittel zur flächigen Übertragung von Radialkräften sind möglich.

Wie in den Figuren 1b, 2b dargestellt, ist das Kontaktelement 12 breiter als der zugehörige Steg 10. Dabei ragen die Seitenkanten des Kontaktelements 12 über das Stegprofil hinaus, so dass die für die Kraftübertragung wirksame Kontaktfläche des einzelnen Stegs 10 sowie des Stents insgesamt vergrößert wird. Bei dem Ausführungsbeispiel gemäß den Figuren 1b, 2b ist das Kontaktelement 12 symmetrisch bzw. mittig zum Steg 10 angeordnet. Die Seitenkanten des Kontaktelements 12 stehen dabei um denselben Betrag über den Steg 10 seitlich vor (T-Form). Andere Anordnungen des Kontaktelements 12 bezüglich des Stegs 10 sind möglich, bspw. ein L-förmiger Querschnitt von Kontaktelement 12 und Steg 10 oder Zwischenformen, bei denen das Kontaktelement 12 auf der einen Stegseite mehr als auf der anderen Stegseite übersteht.

Die Kontaktelemente können um 10% bis 500% breiter als die zugehörigen Stege 10 sein. Der große Bereich ergibt sich daraus, dass für unterschiedliche Anwendungsfälle verschiedene Übermaße der Kontaktelemente 12 zu guten Resultaten im Hinblick auf die Vergrößerung der Kontaktfläche und die gute Implantierbarkeit des Stents führen. Die jeweils geeigneten Unterbereiche bestimmt der Fachmann entsprechend der an den Stent gerichteten Anforderungen. Ein für die Breite der Kontaktelemente besonders geeigneter Bereich liegt zwischen 50 µm und 300 µm.

Im Hinblick auf eine gute Implantierbarkeit des Stents sind die Kontaktelemente 12 flexibel ausgebildet. Die Flexibilität der Kontaktelemente 12 dient dazu, die durch die Kontaktelemente 12 gebildete Kontaktfläche veränderlich zu gestalten derart, dass die Kontaktfläche im gecrimpten Zustand kleiner als im expandierten Zustand ist. Die Veränderlichkeit der Kontaktfläche kann dadurch erreicht werden, dass die Kontaktelemente 12 aus einem Formgedächtnismaterial hergestellt sind, das entsprechend konditioniert ist.

Bei der Auslegung des Stents ist also sowohl die Flexibilität der Kontaktelemente 12 bzw. Folien, als auch deren Festigkeit wichtig. Die Flexibilität ermöglicht die Verformung in den zusammengefalteten Zustand. Bei einer hohen Flexibilität und daher guten Verformbarkeit können breitere Kontaktelemente 12 bzw. Folien den Raum zwischen den Stegen 10 füllen. Die Kontaktelemente 12 bzw. Folien weisen überdies eine ausreichende Festigkeit auf, um die Kraft auf die Gefäßwand zu übertragen.

Die Flexibilität der Kontaktelemente 12 bzw. Folien hat überdies Vorteile im Hinblick auf die Verteilung der Kräfte im Bereich der Stege gegenüber herkömmlichen Stents, bei denen am Rand des Steges höhere Drücke entstehen können als in der Mitte des Steges (Fig. 1a). Dies liegt an der Geometrie der Stege, die dem abgerundeten Profil des Gefäßes nicht folgen und daher an sehr kleinen Kontaktstellen, z.B. an der Kante der Stege, höhere örtliche Drücke ausüben. Demgegenüber liegen die aufgrund ihrer geringeren Wandstärke deutlich flexibleren Kontaktelemente 12 bzw. Folien an der Gefäßwand an, so dass es praktisch zu keiner Bildung von Spannungsspitzen kommt, sondern der Druck gleichmäßig auf die Gefäßwand ausgeübt wird.

Die Höhe bzw. Wandstärke des Kontaktelements 12 (KE) beträgt zwischen 0,5 µm und 100 µm bzw. allgemein zwischen 0,5% und 50% der Höhe des jeweils zugeordneten Stegs 10 (GE). Geeignete Unterbereiche wählt der Fachmann in Abhängigkeit von der Stentgröße und/oder der gewünschten Stabilität bzw. Flexibilität der Kontaktelemente 12 und/oder dem Gewicht des Stents. Bei Neurostents, d.h. Stents zur Implantation in kleinere, cerebrale Blutgefäße, beträgt die Höhe der Stege 10 (GE) im Allgemeinen zwischen 50 µm und 90 µm. Die Höhe der Kontaktelemente 12 (KE) kann demnach beispielsweise zwischen 5 µm und 9 µm betragen, bei einem Verhältnis von 1:10 (KE:GE).

Bei der Verwendung von Folien zur Bildung der Kontaktelemente 12 sind insgesamt folgende Eigenschaften zu berücksichtigen. Die Folie weist eine ausreichend geringe Wandstärke auf, um den Gesamtdurchmesser des Systems innerhalb des Katheters nicht wesentlich zu erhöhen. Die Steifigkeit der Folie ist angepasst, um einen Druck auf die Gefäßwand auszuüben. Die Flexibilität der Folie lässt ein Zusammenfalten des Stents innerhalb des Katheters zu. Die Verbindung der Folie mit den Stegen 12 ist für die üblicherweise zu erwartende Beanspruchung ausgelegt. Die Folie ist biokompatibel.

Im Hinblick auf diese Eigenschaften sind dünne Metallfolien, bspw. aus NiTi-Legierungen, insbesondere Nitinol, für die Kontaktelemente 12 besonders geeignet. Derartige Metallfolien weisen eine erhebliche Festigkeit auch bei sehr dünnen Wandstärken auf.

Einen Gesamtquerschnitt durch einen Stent im implantierten Zustand zeigt Fig. 2a, wobei die Kontaktelemente 12 aus Gründen der Übersichtlichkeit nicht dargestellt sind. Der Stent weist über den Umfang gleichmäßig verteilte Stege 10 auf, die mit ihren jeweils zugeordneten Kontaktelementen 12 (nicht dargestellt) derart an der Gefäßwand 20 anliegen, dass sich der Stent im Wesentlichen der Kontur des Blutgefäßes anpasst. Die Stege 10 begrenzen jeweils Zellen 11, die in der Querschnittsdarstellung als Freiräume dargestellt sind. Fig. 2b zeigt einen Querschnitt durch zwei benachbarte Stege 10 mit einem jeweils zugeordneten Kontaktelement 12. Im implantierten Zustand weist die Zelle 11 zwischen den Stegen 10 eine relativ große Erstreckung in Umfangsrichtung auf, so dass die Kontaktelemente 12 voneinander beabstandet sind.

Liegt der Stent im komprimierten Zustand vor, weist die Zelle 11 eine relativ geringe Erstreckung in Umfangsrichtung auf, so dass die Kontaktelemente 12 sich berühren (Fig. 3b). Die Stege 10 sowie die Kontaktelemente 12 rücken bei der Komprimierung des Stents näher zusammen, so dass sich ein minimaler Querschnittsdurchmesser des gesamten Stents ergibt (Fig. 3a). Auf diese Weise kann der Stent in einen Katheter 30 eingebracht werden, der einen kleineren Querschnittsdurchmesser aufweist, als das Blutgefäß, in das der Stent implantiert wird. Aus Gründen der Übersichtlichkeit zeigt auch Fig. 3a keine Kontaktelemente 12.

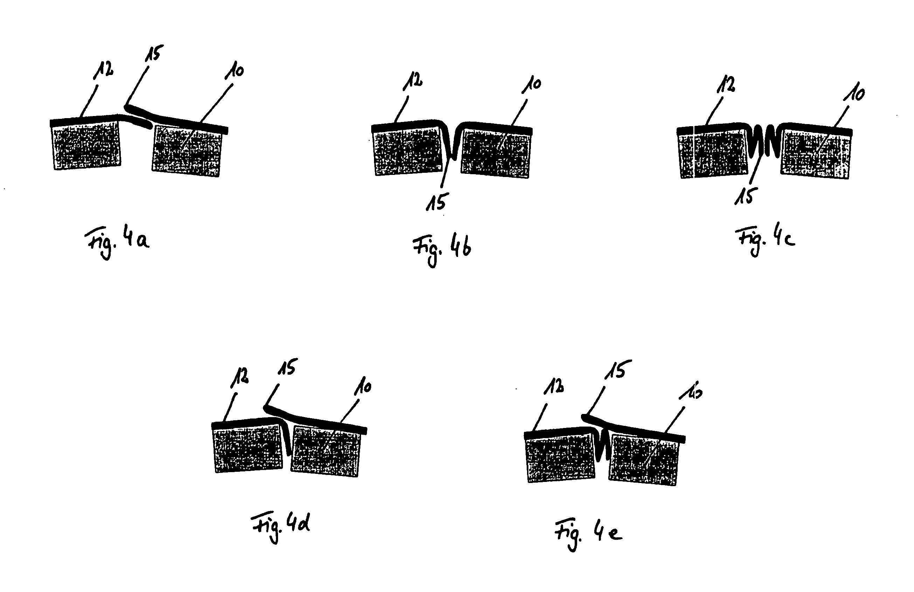

In den Figuren 4a bis 4e sind verschiedene Ausführungsbeispiele von Stents dargestellt, die durch Verformung der flexiblen Kontaktelemente 12 bzw. Folien im komprimierten Zustand des Stents gekennzeichnet sind:
Gemäß Fig. 4a sind die Kontaktelemente 12 so angeordnet bzw. angepasst, dass deren über das Stegprofil vorstehende Bereiche sich im komprimierten Zustand zumindest teilweise überlappen. Beim Crimpen schiebt sich dabei ein Kontaktelement 12 über ein benachbartes Kontaktelement 12. Hierfür ist eine vergleichsweise geringe Flexibilität der Kontaktelemente 12, bzw. Folien erforderlich, da die Kontaktelemente 12, bzw. Folien nur in radialer Richtung um einen geringen Betrag ausgelenkt werden. Der Abstand zwischen den Stegen 10 im komprimierten Zustand entspricht hierbei in etwa dem Übermaß, um das ein Kontaktelement 12 über das Stegprofil vorsteht. Wenn zusätzlich eine Verformung zumindest eines der Kontaktelemente 12, bzw. Folien in tangentialer Richtung erfolgt, kann der Stegabstand weiter verringert werden.

Die Kontaktelemente 12 können derart angeordnet bzw. angepasst sein, dass sich diese bei der Komprimierung verformen und radial nach innen biegen (Fig. 4b). Der überstehende Bereich wenigstens eines Kontaktelements 12 ragt dabei in die zugehörige Zelle 11 hinein. Die Kontaktelemente 12 stehen dabei höchstens um einen Betrag über den jeweils zugeordneten Steg 10 hinaus bzw. sind höchstens um einen Betrag umgebogen, der etwa der Höhe des Stegs 10 entspricht, um zu vermeiden, dass die Enden der umgebogenen Kontaktelemente 12 in den Strömungsquerschnitt ragen.

Gemäß Fig. 4c sind die Kontaktelemente 12 zwischen benachbarten Stegen 10 in tangentialer Richtung verformt, insbesondere gefaltet, so dass der Bereich zwischen den Stegen 10 im komprimierten Zustand durch eine im Querschnitt im Wesentlichen ziehharmonikaförmige Anordnung der Kontaktelemente 12 ausgefüllt ist. Dabei stoßen die Enden bzw. Längskanten der Kontaktelemente 12 an die Seitenflächen der Stege 10.

Kombinationen der vorgenannten Anordnungen der Kontaktelemente 12 im komprimierten Zustand sind möglich. Beispielsweise kann ein Kontaktelement 12 zwischen zwei Stegen 10 radial nach innen gebogen sein und ein benachbartes bzw. gegenüber liegendes Kontaktelement 12 das nach innen gebogene Kontaktelement 12 überlappen (Fig. 4d). Gemäß Fig. 4e ist ein Kontaktelement 12 zwischen benachbarten Stegen 10 in tangentialer Richtung verformt, insbesondere gefaltet, wobei das solchermaßen verformte Kontaktelement 12 von einem weiteren Kontaktelement 12 radial außen überlappt ist.

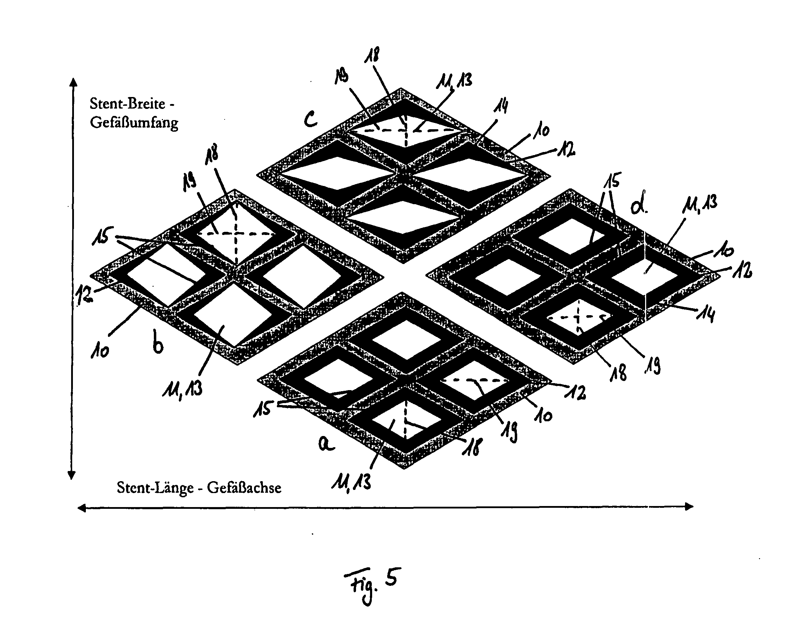

Die Fig. 5a-d zeigen jeweils eine Draufsicht auf verschiedene Zellen 11, aus denen jeweils ein Stent aufgebaut ist oder die in einem Stent miteinander kombiniert sind. Dabei sind die Zellen der Fig. 5a-d jeweils gleich groß und durch Stege 10 begrenzt. Auf den Stegen 10 sind Kontaktelemente 12 angeordnet, wobei die Darstellung derart gewählt ist, dass die Blickrichtung im Wesentlichen von unten bzw. vom Inneren des Stents nach außen gerichtet ist, so dass sowohl die Stege 10, als auch die auf dem Außenumfang angeordneten Kontaktelemente 12 bzw. Folien zu erkennen sind, die seitlich über die Stege 10 vorstehen. Die Zellen 11 weisen eine Rautenform auf, deren längere Diagonale sich in Längsrichtung des Stents erstreckt und deren kürzere Diagonale sich in Umfangsrichtung des Stents erstreckt.

Bei der Ausführungsform gemäß Fig. 5a begrenzen die Kontaktelemente 10 eine ebenfalls rautenförmige Öffnung 13, die der Kontur der Zelle 11 angepasst ist bzw. dieser entspricht. Die einzelnen Kontaktelemente 12 umfassen im Wesentlichen rechtwinklige Streifen, insbesondere Folienstreifen, die mittig auf dem jeweiligen Steg 10, bzw. achsensymmetrisch zum Steg 10 in dessen Längsrichtung angeordnet sind. Dabei ragen die Kontaktelemente 12 auf beiden Seiten der Stege 10 jeweils um denselben Betrag über diese hinaus, so dass die Längskanten der Kontaktelemente sowohl parallel zueinander, als auch parallel zum Steg 10 sind.

Beispielsweise sind die Kontaktelemente 12 gemäß Fig. 5a um ca. 300 % breiter als die Stege 10, so dass sie jeweils um die Breite des Steges 10 auf jeder Seite des Steges über das Stegprofil vorstehen.

Die Kontaktelemente 12 gemäß Fig. 5b sind ebenfalls breiter als die Stege 10. Im Unterschied zu dem Ausführungsbeispiel gemäß Fig. 5a sind die streifenförmigen Kontaktelemente 12 schräg, insbesondere diagonal auf den Stegen 10 angeordnet. Dabei schließt eine erste Längskante 15 des Kontaktelements 12 mit einem ersten Längsende des Stegs 10 ab. Die gegenüber angeordnete zweite Längskante 15 des Kontaktelements 12 schließt mit dem anderen zweiten Längsende 14 des Stegs 10 ab (Abstand zwischen Steg 10 und Längskante 15 jeweils = 0). In Längsrichtung des Stegs 10 erhöht sich der Abstand zwischen dem Steg 10 und der ersten Längskante 15 des Kontaktelements 12, während sich der Abstand zwischen dem Steg 10 und der zweiten Längskante 15 in gleicher Richtung verringert. Dadurch wird bewirkt, dass die Öffnung 13, die durch die Kontaktelemente 12 begrenzt wird, eine in Längsrichtung des Stents kürzere Diagonale 19 aufweist, als die jeweils zugeordnete Zelle 11 und die Diagonale 18 in Umfangsrichtung gleich groß ist wie die Diagonale der Zelle 11.

Die über die Stege 10 hinausragenden Seiten der Kontaktelemente 12 können demnach eine dreieckige Form aufweisen, wobei die beiden durch einen Steg 10 getrennten, dreieckförmigen Seiten jeweils versetzt, insbesondere punktsymmetrisch zur Mitte des Stegs 10, angeordnet sein können (Fig. 5b, 5c). Der Abstand der einen Längskante 15 des Kontaktelements 12 zum Steg 10 ist in diesem Fall an einem Längsende 14 des Stegs 10 null, während der Abstand der anderen Längskante 15 zum Steg 10 an dem selben Längsende 14 maximal ist. Dabei kann das gesamte Kontaktelement 12 eine im Wesentlichen rechteckige Form aufweisen, wobei das Kontaktelement 12 mit dem Steg 10 entlang einer der Diagonalen der Rechteckform verbunden ist.

Es ist ferner möglich, dass die beiden dreieckförmigen Seiten achsensymmetrisch zu einer Längsachse des Stegs 10 angeordnet sind, so dass der Abstand der beiden Längskanten 15 zueinander an einem Längsende 14 des Stegs 10 der Breite des Stegs 10 entspricht und am gegenüberliegenden Längsende 14 größer als die Stegbreite ist. Die Kontaktelemente 12, insbesondere die die Stegbreite überragenden Seiten der Kontaktelemente 12, können auch andere Formen anstelle der Dreiecksform aufweisen. Beispielsweise können die die Stege 10 überragenden Seiten im Wesentlichen rechteckig ausgebildet sein (Fig. 5a. 5d).

Fig. 5c zeigt ein Ausführungsbeispiel, bei dem die Kontaktelemente 12 ebenfalls schräg bzw. diagonal auf den Stegen 10 angeordnet sind, wie bereits zu Fig. 5b beschrieben, wobei die rautenförmige Öffnung 13 jedoch derart ausgebildet ist, dass die Längsdiagonale 19, d.h. die Diagonale der Rautenform in Längsrichtung des Stents, genau so lang ist wie die Längsdiagonale der Zelle 11 und die Umfangsdiagonale 18, d.h. die Diagonale der Rautenform in Umfangsrichtung des Stents, kürzer als die Umfangsdiagonale der Zelle 11 ist. Im Unterschied zu den Kontaktelementen gemäß Fig. 5a und b sind die Kontaktelemente im Bereich der Umfangsdiagonalen 18 der Zelle 11 nicht miteinander verbunden, sondern weisen eine Schnittstelle 14 auf, so dass sich die Kontaktelemente 12 in diesem Bereich voneinander entfernen können, wenn der Stent in den komprimierten Zustand überführt wird. Bei der Komprimierung des Stents verlängert sich die Längsdiagonale der Zelle 11, wohingegen die Umfangsdiagonale der Zelle 11 kleiner wird. Dadurch werden die benachbarten Kontaktelemente 12 im Bereich der Umfangsdiagonalen der Zelle 11 auseinander gezogen. Durch die Schnittstelle 14 wird dabei erreicht, dass die Kontaktelemente 12 nur leicht bzw. nicht gedehnt werden.

Fig. 5d zeigt eine Anordnung von Kontaktelementen 12 auf Gitterelementen bzw. Stegen 10, wobei die von den Kontaktelementen 12 begrenzte Öffnung 13 rautenförmig an die Kontur der rautenförmigen Zelle 11 angepasst ist. Insofern entspricht dieses Ausführungsbeispiel der Darstellung gemäß Fig. 5a. Der Unterschied zu dem Ausführungsbeispiel gemäß Fig. 5a besteht darin, dass die Kontaktelemente 12 gemäß Fig. 5d mehrere Schnittstellen 14 aufweisen, die sich im Wesentlichen senkrecht von den jeweils zugeordneten Stegen 10 entlang der Längskanten 15 der Kontaktelemente 12 erstrecken. Dabei erstrecken sich die Schnittstellen 14 einer Zelle 11 im Wesentlichen in derselben Richtung. Dadurch können sich die Kontaktelemente 12 beim Komprimieren des Stents übereinander schieben, so dass die Kontaktelemente 12 im komprimierten Zustand überlappend angeordnet sind.

Im Allgemeinen sind für den erfindungsgemäßen Stent Stegbreiten von ca. 25-35 µm, insbesondere 30 µm, vorgesehen. Die Höhe der Stege 10 kann zwischen 50 und 90 µm, insbesondere 70 µm, betragen. Dabei beträgt der maximale Abstand der Stege 10 vorzugsweise maximal 500 µm, insbesondere das zehnfache der Stegbreite. Das Verhältnis der Breite der Kontaktelemente 12 zur Breite der Gitterelemente 10 beträgt vorzugsweise maximal 500 %, d.h. dass die Kontaktelemente 12 auf beiden Seiten der Gitterelemente 10 um den Betrag der doppelten Breite des Gitterelements bzw. Stegs 10 über den Steg 10 hinausragen. Dieses Ausführungsbeispiel bezieht sich auf einen Stent zur Implantation in kleine Gefäße, insbesondere cerebrale Gefäße, wobei die Erfindung nicht darauf beschränkt ist. Für den Einsatz der erfindungsgemäßen Stents in andere, insbesondere größere, Gefäße wählt der Fachmann im Allgemeinen andere Größen und/oder Größenverhältnisse.

Mit dem vorstehend beschriebenen Stent wird erreicht, dass der gesamte Umfang des Stents im implantierten Zustand über das bisher aus dem Stand der Technik bekannte Maß vergrößert werden kann, wobei die lokalen Radialkräfte F, die im implantierten Zustand auf die Gefäßwand wirken, minimiert werden. Das wird insbesondere dadurch erreicht, dass die im implantierten Zustand wirksame Kontaktfläche erhöht wird, woraus eine gleichmäßige Verteilung der Radialkräfte F resultiert.

Mit dem erfindungsgemäßen Stent wird also sowohl eine hohe Steganzahl, als auch eine hohe Stegbreite erzielt. Daraus resultiert eine Erhöhung des gesamten Umfangs des Stents und somit eine Verringerung des lokal auf die Gefäßwand 20 wirkenden Drucks. Insgesamt kann der Umfang des erfindungsgemäßen Stents trotz eines relativ großen Umfangs bzw. einer relativ großen Kontaktfläche im implantierten Zustand auf einen kleinen Umfang komprimiert bzw. gecrimpt werden, so dass der Stent mit Hilfe eines handelsüblichen Zufuhrsystems, insbesondere Katheters, implantiert werden kann. Das bedeutet, dass die im implantierten Zustand wirksame Kontaktfläche des Stents gegenüber bisher bekannten Stents, die im komprimierten Zustand den gleichen Querschnittsdurchmesser aufweisen, erhöht ist.

Eine mögliche Technik zum Auftragen bzw. Aufbringen der Kontaktelemente 12, insbesondere aus einer Nickel-Titan-Legierung wie beispielsweise Nitinol, auf die Gitterelemente 10 ist das Sputtern. Dabei werden Ionen auf die Oberfläche des Stents geschossen. Es ist möglich, den besonderen Stegquerschnitt (T- oder L-Form) integral, d.h. die Kontaktelemente 12 und die Stege 10 einstückig herzustellen, wobei die Herstellung vorzugsweise durch einstückiges Sputtern der Stege 10 und Kontaktelemente 12 erfolgt. Es ist auch möglich, die Kontaktelemente 12 direkt auf die Stege 10 einer zuvor hergestellten Gitterstruktur aufzusputtern. Generell können die Kontaktelemente 12 ihre Form entweder bereits während des Sputterverfahrens erhalten oder in einem weiteren Schritt beispielsweise durch ein Ätzverfahren geformt werden. Im zweiten Fall werden die Kontaktelemente 12 bzw. die Folie zunächst auf den gesamten Umfang des Stents aufgebracht und anschließend die Öffnungen 13 freigeätzt. Ein geeignetes Ätzverfahren ist beispielsweise in der auf die Anmelderin zurückgehenden DE 10 2006 029 831 A1 offenbart.

Besonders vorteilhaft ist es, wenn das erfindungsgemäße Verfahren die Verwendung zumindest einer Opferschicht umfasst, so dass eine schnelle, serientaugliche Herstellung hochgenauer Steggeometrien, insbesondere mit hoher Kantengenauigkeit, erreicht wird. Ein zur einstückigen Herstellung eines erfindungsgemäßen Stents geeignetes Verfahren beschreibt beispielsweise die DE 10 2006 007 231 A1, die ebenfalls auf die Anmelderin zurückgeht.

### Bezugszeichenliste

- F: Radialkraft
- 10: Stege
- 11: Zellen
- 12: Kontaktelemente
- 13: Öffnung
- 14: Schnittstelle
- 15: Längskante
- 18: Umfangsdiagonale
- 19: Längsdiagonale
- 20: Gefäßwand

## Patentansprüche

1. Stent mit einer rohrförmigen Gitterstruktur, die Gitterelemente, insbesondere Stege (10), und durch Gitterelemente begrenzte Zellen (11) umfasst, wobei die Gitterstruktur in einen komprimierten Zustand mit relativ kleinerem Querschnittsdurchmesser und in einen expandierten Zustand mit relativ größerem Querschnittsdurchmesser überführbar ist,
**dadurch gekennzeichnet, dass**
den Gitterelementen flexible Kontaktelemente (12) zugeordnet sind, die zur Übertragung von Radialkräften auf eine Gefäßwand (20) angepasst sind und sich auf einem Außenumfang der Gitterstruktur im Wesentlichen in Längsrichtung der jeweils zugeordneten Gitterelemente erstrecken, wobei die Kontaktelemente (12) zumindest abschnittsweise breiter als die jeweils zugeordneten Gitterelemente sind.

2. Stent nach Anspruch 1,
**dadurch gekennzeichnet, dass**
die Gitterelemente jeweils zusammen mit einem zugeordneten Kontaktelement (12) zumindest abschnittsweise einen im Wesentlichen T-förmigen oder L-förmigen Querschnitt bilden.

3. Stent nach Anspruch 1 oder 2,
**dadurch gekennzeichnet, dass**
die Kontaktelemente (12) jeweils um wenigstens 10%, insbesondere wenigstens 15%, insbesondere wenigstens 20%, insbesondere wenigstens 30%, insbesondere wenigstens 40%, insbesondere wenigstens 50%, insbesondere wenigstens 60%, insbesondere wenigstens 70%, insbesondere wenigstens 80%, insbesondere wenigstens 90%, insbesondere wenigstens 100%, breiter sind als das jeweils zugeordnete Gitterelement.

4. Stent nach wenigstens einem der Ansprüche 1 bis 3,
**dadurch gekennzeichnet, dass**
die Kontaktelemente (12) jeweils um höchstens 500%, insbesondere höchstens 400%, insbesondere höchstens 300%, insbesondere höchstens 200%, insbesondere höchstens 100%, breiter sind als das jeweils zugeordnete Gitterelement.

5. Stent nach wenigstens einem der Ansprüche 1 bis 4,
**dadurch gekennzeichnet, dass**
die Breite eines oder mehrerer Kontaktelemente (12) über den Umfang und/oder die Länge des Stents variabel ist.

6. Stent nach wenigstens einem der Ansprüche 1 bis 5,
**dadurch gekennzeichnet, dass**
die Zellen (11) der Gitterstruktur jeweils eine Öffnung (13) aufweisen, die durch die Kontaktelemente (12) der Gitterelemente begrenzt ist, die die jeweilige Zelle (11) bilden.

7. Stent nach Anspruch 6,
**dadurch gekennzeichnet, dass**
die Öffnungen (13) jeweils an die Kontur der Zelle (11) angepasst sind.

8. Stent nach Anspruch 6 oder 7,
**dadurch gekennzeichnet, dass**
die Öffnungen (13) im Wesentlichen rautenförmig sind.

9. Stent nach wenigstens einem der Ansprüche 1 bis 8,
**dadurch gekennzeichnet, dass**
die Kontaktelemente (12) jeweils wenigstens eine Längskante (15) aufweisen, die sich parallel zu einer Längsachse des jeweils zugeordneten Gitterelements erstreckt.

10. Stent nach wenigstens einem der Ansprüche 1 bis 9,
**dadurch gekennzeichnet, dass**
die Kontaktelemente (12) jeweils wenigstens eine Längskante (15) aufweisen, die in einem Winkel zur Längsachse des jeweils zugeordneten Gitterelements angeordnet ist.

11. Stent nach wenigstens einem der Ansprüche 1 bis 10,
**dadurch gekennzeichnet, dass**
die Kontaktelemente (15) jeweils zwei Längskanten (15) aufweisen, die zueinander parallel und/oder zu einer Längsachse des jeweils zugeordneten Gitterelements in einem Winkel angeordnet sind.

12. Stent nach wenigstens einem der Ansprüche 1 bis 11,
**dadurch gekennzeichnet, dass**
die Kontaktelemente (12) im Wesentlichen rechteckige Streifen bilden, die im Wesentlichen diagonal zum jeweils zugeordneten Gitterelement angeordnet sind.

13. Stent nach wenigstens einem der Ansprüche 1 bis 12,
**dadurch gekennzeichnet, dass**
die Kontaktelemente (12) in Eckbereichen der Zellen (11) im expandierten Zustand überlappend und/oder aneinander angrenzend angeordnet oder miteinander verbunden sind.

14. Stent nach wenigstens einem der Ansprüche 1 bis 13,
**dadurch gekennzeichnet, dass**
die Kontaktelemente (12) eine Höhe zwischen 0,5 µm und 100 µm, insbesondere zwischen 1 µm und 50 µm, insbesondere zwischen 2 µm und 30 µm, insbesondere zwischen 3 µm und 20 µm, insbesondere zwischen 5 µm und 10 µm, aufweisen.

15. Stent nach wenigstens einem der Ansprüche 1 bis 14,
**dadurch gekennzeichnet, dass**
die Höhe der Kontaktelemente (12) wenigstens 0,5%, insbesondere wenigstens 1%, insbesondere wenigstens 2%, insbesondere wenigstens 5%, insbesondere wenigstens 10%, der Höhe des jeweils zugeordneten Gitterelements entspricht.

16. Stent nach wenigstens einem der Ansprüche 1 bis 15,
**dadurch gekennzeichnet, dass**
die Höhe der Kontaktelemente (12) höchstens 50%, insbesondere höchstens 40%, insbesondere höchstens 30%, insbesondere höchstens 20%, insbesondere höchstens 15%, insbesondere höchstens 10%, der Höhe des jeweils zugeordneten Gitterelements entspricht.

17. Stent nach wenigstens einem der Ansprüche 1 bis 16,
**dadurch gekennzeichnet, dass**
die Höhe eines und/oder mehrerer Kontaktelemente (12) über den Umfang und/oder die Länge des Stents variabel ist.

18. Stent nach wenigstens einem der Ansprüche 1 bis 17,
**dadurch gekennzeichnet, dass**
die Höhe eines und/oder mehrerer Kontaktelemente (12) über die Breite und/oder die Länge der Kontaktelemente (12) variabel ist.

19. Stent nach wenigstens einem der Ansprüche 1 bis 18,
**dadurch gekennzeichnet, dass**
ein und/oder mehrere Kontaktelemente (12) im Bereich des jeweils zugeordneten Gitterelements eine größere Höhe aufweisen als in einem Außenbereich.

20. Stent nach wenigstens einem der Ansprüche 1 bis 19,
**dadurch gekennzeichnet, dass**
die Kontaktelemente (12) ein Formgedächtnismaterial, insbesondere eine Nickel-Titan-Legierung, umfassen.

21. Stent nach wenigstens einem der Ansprüche 1 bis 20,
**dadurch gekennzeichnet, dass**
die Kontaktelemente (12) eine strukturierte Oberfläche, insbesondere Poren oder Rillen, aufweisen.

22. Verfahren zum Herstellen eines Stents gemäß Anspruch 1, bei dem flexible Kontaktelemente (12) mit Gitterelementen verbunden oder zusammen mit den Gitterelementen einstückig hergestellt werden derart, dass einem Gitterelement jeweils wenigstens ein Kontaktelement (12) zugeordnet ist, das über den Querschnitt des Gitterelements hinausragt.

23. Verfahren nach Anspruch 22,
**dadurch gekennzeichnet, dass**
die Kontaktelemente (12) durch Laserstrahl-Mikroschweißen mit den Gitterelementen verbunden werden.

24. Verfahren nach Anspruch 22,
**dadurch gekennzeichnet, dass**
die Kontaktelemente (12) mit den Gitterelementen verklebt werden.

25. Verfahren nach Anspruch 22,
**dadurch gekennzeichnet, dass**
die Kontaktelemente (12) durch Sputtern, insbesondere Magnetronsputtern oder Ionenstrahlsputtern, hergestellt werden.

## Claims

1. Stent having a tubular lattice structure, the lattice elements, in particular webs (10), and cells (11) delimited by the lattice elements, wherein the lattice structure may be transferred into a compressed state having a relatively smaller cross-sectional diameter and into an expanded state having a relatively larger cross-sectional diameter, **characterised in that**
flexible contact elements (12) are associated with the lattice elements, said contact elements being adapted for transferring radial forces onto a vessel wall (20) and extending on the outer circumference of the lattice structure substantially in the longitudinal direction of each associated lattice element, wherein the contact elements (12) are wider in each case than the associated lattice elements, at least in sections.

2. Stent according to claim 1,
**characterised in that**
the lattice elements in each case together with an associated contact element (12) form a substantially T-shaped or L-shaped cross-section, at least in sections.

3. Stent according to claim 1 or 2,
**characterised in that**
the contact elements (12) are in each case wider than each associated lattice element by at least 10%, in particular at least 15%, in particular at least 20%, in particular at least 30%, in particular at least 40%, in particular at least 50%, in particular at least 60%, in particular at least 70%, in particular at least 80%, in particular at least 90%, in particular at least 100%.

4. Stent according to one of claims 1 to 3,
**characterised in that**
the contact elements (12) are in each case wider than each associated lattice element by at most 500%, in particular at most 400%, in particular at most 300%, in particular at most 200%, in particular at most 100%.

5. Stent according to one of claims 1 to 4,
**characterised in that**
the width of one or a plurality of contact elements (12) is variable over the circumference and/or the length of the stent.

6. Stent according to one of claims 1 to 5,
**characterised in that**
the cells (11) of the lattice structure each have an opening (13), which is delimited by the contact elements (12) of the lattice elements that form each cell (11).

7. Stent according to claim 6,
**characterised in that**
the openings (13) are adapted in each case to the contour of the cell (11).

8. Stent according to claim 6 or 7,
**characterised in that**
the openings (13) are substantially diamond-shaped.

9. Stent according to one of claims 1 to 8,
**characterised in that**
the contact elements (12) each have at least one longitudinal edge (15) which extends parallel to a longitudinal axis of each associated lattice element.

10. Stent according to one of claims 1 to 9,
**characterised in that**,
the contact elements (12) each have at least one longitudinal edge (15) which is disposed at an angle to the longitudinal axis of each associated lattice element.

11. Stent according to one of claims 1 to 10,
**characterised in that**,
the contact elements (12) each have two longitudinal edges (15) which are disposed parallel to one another and/or are disposed at an angle to a longitudinal axis of each associated lattice element.

12. Stent according to one of claims 1 to 11,
**characterised in that**,
the contact elements (12) form substantially rectangular strips which are substantially disposed diagonally to each associated lattice element.

13. Stent according to one of claims 1 to 12,
**characterised in that**,
the contact elements (12) are joined so as to be overlapping and/or adjacent to each other in corner regions of the cells (11) or are joined to each other.

14. Stent according to one of claims 1 to 13,
**characterised in that**,
the contact elements (12) have a height between 0.5 µm and 100 µm, in particular between 1 µm and 50 µm, in particular between 2 µm and 30 µm, in particular between 3 µm and 20 µm, in particular between 5 µm and 10 µm.

15. Stent according to one of claims 1 to 14,
**characterised in that**,
the height of the contact elements (12) corresponds to at least 0.5%, in particular at least 1%, in particular at least 2%, in particular at least 5%, in particular at least 10% of the height of each associated lattice element.

16. Stent according to one of claims 1 to 15,
**characterised in that**,
the height of the contact elements (12) corresponds to at most 50%, in particular at most 40%, in particular at most 30%, in particular at most 20%, in particular at most 15%, in particular at most 10% of the height of each associated lattice element.

17. Stent according to one of claims 1 to 16,
**characterised in that**,
the height of one and/or a plurality of contact elements (12) is variable over the circumference and/or the length of the stent.

18. Stent according to one of claims 1 to 17,
**characterised in that**,
the height of one and/or a plurality of contact elements (12) is variable over the width and/or the length of the contact elements (12).

19. Stent according to one of claims 1 to 18,
**characterised in that**,
one and/or a plurality of contact elements (12) has a greater height in the region of each associated lattice element than in an outer region.

20. Stent according to one of claims 1 to 19,
**characterised in that**,
the contact elements (12) comprise a shape memory material, in particular a nickel-titanium alloy.

21. Stent according to one of claims 1 to 20,
**characterised in that**,
the contact elements (12) have a structured surface, in particular pores or grooves.

22. Method for the production of a stent according to claim 1, in which flexible contact elements (12) are joined with lattice elements or are produced integrally with the lattice elements in such a manner that at least one contact element (12) is associated in each case with a lattice element, the contact element protruding beyond the lattice element's cross-section.

23. Method according to claim 22,
**characterised in that**,
the contact elements (12) are joined to the lattice elements by means of laser beam micro welding.

24. Method according to claim 22,
**characterised in that**,
the contact elements (12) are bonded to the lattice elements.

25. Method according to claim 22,
**characterised in that**,
the contact elements (12) are produced by means of sputtering, in particular by means of magnetron sputtering or ion beam sputtering.

## Revendications

1. Prothèse présentant une structure en treillis tubulaire qui comprend des éléments de treillis, en particulier des montants (10) et des cellules (11) limitées par des éléments de treillis, la structure en treillis pouvant se transformer en un état comprimé présentant un diamètre transversal relativement inférieur et en un état déployé présentant un diamètre transversal relativement plus grand,
**caractérisé en ce que**
des éléments de contact flexibles (12) qui sont adaptés à la transmission de forces radiales sur une paroi vasculaire (20) et qui s'étendent sur un pourtour externe de la structure en treillis essentiellement dans le sens de la longueur des éléments en treillis attribués respectivement, sont attribués aux éléments de treillis, les éléments de contact (12) étant au moins partiellement plus larges que les éléments de treillis attribués.

2. Prothèse selon la revendication 1,
**caractérisé en ce que**
les éléments de treillis forment respectivement, conjointement avec un élément de contact (12) attribué, au moins partiellement une section essentiellement en forme de T ou en forme de L.

3. Prothèse selon la revendication 1 ou 2,
**caractérisé en ce que**
les éléments de contact (12) sont respectivement plus larges d'au moins 10 %, en particulier d'au moins 15 %, en particulier d'au moins 20 %, en particulier d'au moins 30 %, en particulier d'au moins 40 %, en particulier d'au moins 50 %, en particulier d'au moins 60 %, en particulier d'au moins 70 %, en particulier d'au moins 80 %, en particulier d'au moins 90 %, en particulier d'au moins 100 % que l'élément de treillis respectivement attribué.

4. Prothèse selon au moins l'une des revendications 1 à 3,
**caractérisé en ce que**
les éléments de contact (12) sont respectivement plus larges d'au plus 500 %, en particulier d'au plus 400 %, en particulier d'au plus 300 %, en particulier d'au plus 200 %, en particulier d'au plus 100 % que l'élément de treillis respectivement attribué.

5. Prothèse selon au moins l'une des revendications 1 à 4,
**caractérisé en ce que**
la largeur d'un ou plusieurs éléments de contact (12) est variable sur le pourtour et/ou la longueur du prothèse.

6. Prothèse selon au moins l'une des revendications 1 à 5,
**caractérisé en ce que**
les cellules (11) de la structure de treillis présentent respectivement une ouverture (13) qui est limitée par les éléments de contact (12) des éléments de treillis qui forment la cellule respective (11).

7. Prothèse selon la revendication 6,
**caractérisé en ce que**
les ouvertures (13) sont adaptées respectivement au contour de la cellule (1).

8. Prothèse selon la revendication 6 ou 7,
**caractérisé en ce que**
les ouvertures (13) sont essentiellement en forme de losange.

9. Prothèse selon au moins l'une des revendications 1 à 8,
**caractérisé en ce que**
les éléments de contact (12) présentent respectivement au moins un bord longitudinal (15) qui s'étend parallèlement à un axe longitudinal de l'élément de treillis respectivement attribué.

10. Prothèse selon au moins l'une des revendications 1 à 9,
**caractérisé en ce que**
les éléments de contact (12) présentent respectivement au moins un bord longitudinal (15) qui est disposé dans un angle par rapport à l'axe longitudinal de l'élément de treillis respectivement attribué.

11. Prothèse selon au moins l'une des revendications 1 à 10,
**caractérisé en ce que**
les éléments de contact (12) présentent respectivement deux bords longitudinaux (15) qui sont disposés parallèlement les uns par rapport aux autres et/ou par rapport à un axe longitudinal de l'élément de treillis respectivement attribué dans un angle.

12. Prothèse selon au moins l'une des revendications 1 à 11,
**caractérisé en ce que**
les éléments de contact (12) forment essentiellement des bandes rectangulaires qui sont disposées de façon essentiellement en diagonale par rapport à l'élément de treillis respectivement attribué.

13. Prothèse selon au moins l'une des revendications 1 à 12,
**caractérisé en ce que**
les éléments de contact (12) sont disposés dans les zones d'angle des cellules (11) à l'état déployé, de façon chevauchante et/ou de façon directement adjacente ou sont reliés les uns aux autres.

14. Prothèse selon au moins l'une des revendications 1 à 13,
**caractérisé en ce que**
les éléments de contact (12) présentent une hauteur entre 0,5 µm et 100 µm, en particulier entre 1 µm et 50 µm, en particulier entre 2 µm et 30 µm, en particulier entre 3 µm et 20 µm, en particulier entre 5 µm et 10 µm.

15. Prothèse selon au moins les revendications 1 à 14,
**caractérisé en ce que**
la hauteur des éléments de contact (12) correspond à au moins 0,5 %, en particulier au moins 1 %, en particulier au moins 2 %, en particulier au moins 5 %, en particulier au moins 10 %, de la hauteur de l'élément de treillis respectivement attribué.

16. Prothèse selon au moins l'une des revendications 1 à 15,
**caractérisé en ce que**
la hauteur de l'élément de contact (12) correspond à au plus 50 %, en particulier au plus 40 %, en particulier au plus 30 %, en particulier au plus 20 %, en particulier au plus 15 %, en particulier au plus 10 % de la hauteur de l'élément de treillis respectivement attribué.

17. Prothèse selon au moins l'une des revendications 1 à 16,
**caractérisé en ce que**
la hauteur d'un et/ou de plusieurs éléments de contact (12) est variable sur le pourtour et/ou la longueur du prothèse.

18. Prothèse selon au moins l'une des revendications 1 à 17,
**caractérisé en ce que**
la hauteur d'un et/ou plusieurs éléments de contact (12) est variable sur la largeur et/ou la longueur des éléments de contact (12).

19. Prothèse selon au moins l'une des revendications 1 à 18,
**caractérisé en ce**
**qu'**un et/ou plusieurs éléments de contact (12) présentent dans la zone de l'élément de treillis respectivement attribué, une hauteur supérieure à celle dans une zone extérieure.

20. Prothèse selon au moins l'une des revendications 1 à 19,
**caractérisé en ce que**
les éléments de contact (12) comprennent un matériau à mémoire de forme, en particulier un alliage nickel-titane.

21. Prothèse selon au moins l'une des revendications 1 à 20,
**caractérisé en ce que**
les éléments de contact (12) présentent une surface structurée, en particulier des pores ou des sillons.

22. Procédé de production d'un prothèse selon la revendication 1, dans lequel des éléments de contact flexibles (12) sont liés à des éléments de treillis ou sont produits d'un seul tenant conjointement avec les éléments de treillis, de telle sorte qu'à un élément de treillis soit attribué respectivement au moins un élément de contact (12) qui dépasse au-dessus de la section de l'élément de treillis.

23. Procédé selon la revendication 22,
**caractérisé en ce que**
les éléments de contact (12) sont reliés par des micro-soudures à rayon laser aux éléments de treillis.

24. Procédé selon la revendication 22,
**caractérisé en ce que**
les éléments de contact (12) sont collés aux éléments de treillis.

25. Procédé selon la revendication 22, **caractérisé en ce que** les éléments de contact (12) sont produits par pulvérisation cathodique, en particulier pulvérisation cathodique magnétron ou pulvérisation cathodique à rayons ioniques.
